# EUROPÄISCHE PATENTSCHRIFT

(11) **EP 0 677 040 B1**
(45) Veröffentlichungstag und Bekanntmachung des Hinweises auf die Patenterteilung: **27.10.1999**
(21) Anmeldenummer: 94903822.8
(22) Anmeldetag: 17.12.1993
(51) Int. Cl.: C07C 239/20, C07C 259/06, C07C 251/54

(54) **VERFAHREN ZUR HERSTELLUNG VON HYDROXYLAMIN-ETHERN SOWIE DEREN SALZEN UND ZWISCHENPRODUKTE HIERFÜR**
PROCESS AND INTERMEDIATE PRODUCTS FOR PREPARING HYDROXYLAMINE-ETHERS AND THEIR SALTS
PROCEDE ET PRODUITS INTERMEDIAIRES POUR LA PREPARATION D'ETHERS D'HYDROXYLAMINE ET DE LEURS SELS

(30) Priorität: 29.12.1992 DE 4244390
(43) Veröffentlichungstag der Anmeldung: 18.10.1995
(73) Patentinhaber: BASF Aktiengesellschaft, 67063 Ludwigshafen (DE)
(72) Erfinder: HARREUS, Albrecht, D-67063 Ludwigshafen (DE); GÖTZ, Norber, D-67547 Worms (DE); MAYWALD, Volker, D-67069 Ludwigshafen (DE); RANG, Harald, D-67122 Altrip (DE); MISSLITZ, Ulf, D-67433 Neutstadt (DE); KLEIN, Ulrich, D-67117 Limburgerhof (DE)
(86) Internationale Anmeldenummer: EP9303597
(87) Internationale Veröffentlichungsnummer: WO9414757

(56) Entgegenhaltungen:
- EP-A- 0 023 560
- EP-A- 0 184 546
- DE-A- 2 651 083
- US-A- 3 723 429
- HETEROCYCLES Bd. 20, Nr. 5 , 1983 Seiten 839 - 843 B. BAJWA ET AL. 'The chemistry of drugs. III acid hydrolysis of antimalarial 5-alko xy-6,6-dimethyl-5,6-dihydro-s-triazines' in der Anmeldung erwähnt
- JOURNAL OF THE AMERICAN CHEMICAL SOCIETY Bd. 74 , 1952 , WASHINGTON, DC US Seiten 3956 - 3957 P. TRUITT ET AL. 'Antitubercular studies. III. Hydroxylamines and thiosemicarbazones' in der Anmeldung erwähnt

## Beschreibung

Die vorliegende Erfindung betrifft ein Verfahren zur Herstellung von Hydroxylamin-Ethern der Formel I 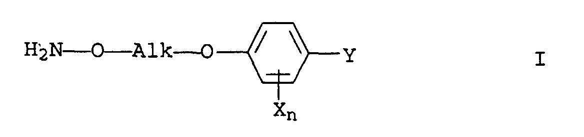 in der die Variablen folgende Bedeutung haben:
- X: Nitro, Cyano, Halogen, C₁-C₄-Alkyl und C₁-C₄-Halogenalkyl;
- Y: Wasserstoff, Nitro, Cyano, Halogen, C₁-C₄-Alkyl und C₁-C₄-Halogenalkyl;
- n: 0-2 oder 1-4 für den Fall, daß Y und alle X Halogen bedeuten;
- Alk: eine C₂- oder C₃-Alkylenkette, die gewünschtenfalls ein bis drei C₁-C₃-Alkylgruppen tragen kann,
sowie deren Salzen mit Mineralsäuren oder starken organischen Säuren.

Die Herstellung der Verbindungen I kann nicht durch direkte O-Alkylierung von Hydroxylamin erfolgen, sondern setzt die Anwendung von Schutzgruppen-Techniken voraus. Solche Methoden zur Synthese von Hydroxylamin-Ethern vom Typ der Verbindungen I werden beispielsweise in Houben-Weyl, Methoden der organischen Chemie, Band E16a, 1990, Seiten 214ff. beschrieben. Aus dieser Literaturstelle ist auch die sogenannte "N-Hydroxyphthalimid-Methode" bekannt, nach der gemäß EP-A 456 112, DE-A 42 04 203 und DE-A 42 04 206 Hydroxylamin-Ether vom Typ der Verbindungen I bisher hergestellt wurden. Für die industrielle Anwendung hat diese Methode jedoch Nachteile. So entstehen bei der Abspaltung der Schutzgruppe neben den gewünschten O-substituierten Hydroxylaminen in der Regel nicht verwertbare Koppelprodukte, beispielsweise Phthalsäurehydrazid bei der Spaltung mit Hydrazin. Eine Rückgewinnung der eingesetzten Schutzgruppe ist normalerweise ebenfalls nicht möglich.

Aus J. Agric. Food. Chem. 38, 514 (1990) ist die Herstellung von Aceton-[O-3-(4-phenoxyphenoxy)propyl] -oxim in Gegenwart von Kalium-tert.-butylat in Dioxan als Lösungsmittel bekannt. Das beschriebene Verfahren eignet sich jedoch nicht für eine industrielle Herstellung der Oximino-Derivate IV, da große Mengen an Lösungsmitteln verbraucht werden. Eine entsprechende Umsetzung mit geringeren Mengen an Lösungsmitteln ist nicht möglich, da in diesem Falle zähe Emulsionen erhalten werden.

Eine weitere Veröffentlichung (J. Am. Chem. Soc., **74**, 3956 (1952)) beschreibt die Kondensation von u.a. 2-Phenoxyethylbromid mit dem Natriumsalz des Acetonoxims - wobei jedoch keinerlei Angaben über die Verfahrensbedingungen gemacht werden - und die Spaltung des erhaltenen Oximethers mit Salzsäure.

In den Druckschriften DE-A 26 51 083, DE-A 26 51 085 und JP-A91/258 757 werden bei Alkylierungsreaktionen mit Hydroxyiminoderivaten vom Typ der Verbindungen II relativ teure, technisch schwierig zu handhabende Basen wie Alkalimetallhydride, beispielsweise Natriumhydrid, Alkalimetallamide, beispielsweise Natriumamid, oder Organometallverbindungen, beispielsweise Butyllithium, verwendet. Hierbei muß wasserfrei gearbeitet werden, was technisch aufwendig ist.

Einige der Oximino-Derivate IV sind bereits aus der U.S. 4,647,698 bekannt {vgl. Formel (B) des Patentes}, und zwar als Pestizide. Zu deren Herstellung aus Hydroxyiminoverbindungen II und Alkylierungsmitteln vom Typ der Verbindungen III wird die Anwesenheit von Natriumhydrid als Base gelehrt. Nachteilig ist hierbei das technisch aufwendige Arbeiten unter Inertgas.

Nach Bioorg. Khim. **12**, 1662 (1986) kann 1-(1-Ethoxyethylidenaminooxy)-2-phenoxyethan durch Umsetzung von 1-(1-Ethoxyethylidenaminooxy)-2-bromethan mit Natriumphenolat in Methanol erhalten werden. Nachteilig ist hierbei jedoch, daß der erstgenannte Reaktionspartner nur in einer Ausbeute von 28% zugänglich ist und daß das Verfahrensprodukt selbst nur mit einer Ausbeute von 40% entsteht.

Außerdem ist der EP-A 023 560 zu entnehmen, daß bestimmte Ketoxime mit (Cyclo)alkyl- oder Arylalkylhalogeniden zu O-substituierten Ketoximen umgesetzt werden können. Die Verwendung von Sulfonsäureestern vom Typ der Verbindungen III als Alkylierungsmittel ist der Druckschrift nicht zu entnehmen.

Bezüglich der Hydrolyse von Oximino-Derivaten IV, in denen R² für C₁-C₄-Alkyl steht, sind der Literatur nur wenige analoge Beispiele zu entnehmen. So erhielten Bajwa et al., Heterocycles **20**, 839 (1983) bei der Hydrolyse einer Verbindung der Formel IV, wobei R¹ und R² Methyl, Alk 1,3-Propyliden und Y Chlor und X₂ 2,5-Dichlor bedeuten, in einem Gemisch aus Salzsäure, Ethanol und Wasser den entsprechende Hydroxylamin-Ether I in Form des Hydrochlorids.

Der vorliegenden Erfindung lag die Aufgabe zugrunde, die Hydroxylamin-Ether I besser zugänglich zu machen.

Demgemäß wurde ein Verfahren zur Herstellung der Hydroxylamin-Ether I sowie deren Salzen mit Mineralsäuren oder starken organischen Säuren gefunden, welches dadurch gekennzeichnet ist, daß man entweder eine Hydroxyiminoverbindung der Formel II 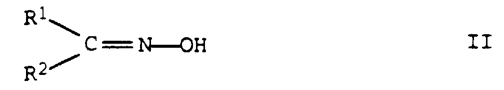 in der R¹ für C₁-C₄-Alkyl, R² für C₁-C₄-Alkyl oder C₁-C₆-Alkoxy oder R¹ und R² zusammen für eine C₄-C₆-Alkylenkette stehen, in Gegenwart von Alkalimetallhydroxid, Alkalimetallalkoholat, Alkalimetallhydrogencarbonat oder Alkalimetallcarbonat als Base, oder das entsprechende Anion von II direkt, mit einem Alkylierungsmittel der Formel III 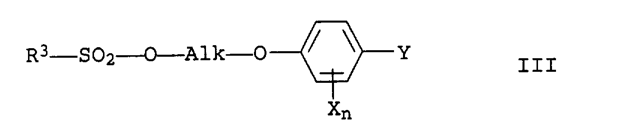 in der R³ für C₁-C₄-Alkyl, C₁-C₄-Halogenalkyl oder gegebenenfalls substituiertes Phenyl steht,
zu einem Oximino-Derivat der Formel IV 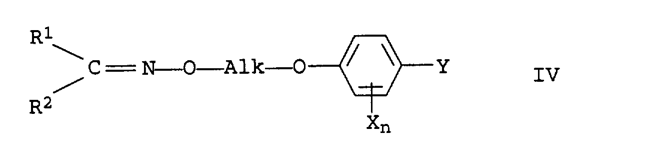 umsetzt,
das Oximino-Derivat IV anschließend mittels Mineralsäure oder einer starken organischen Säure zum entsprechenden Salz von I spaltet und dieses gewünschtenfalls mittels Base in die freie Verbindung I überführt.

Die Reste R¹, R², X, Y und Alk haben im einzelnen die folgenden Bedeutungen:
- R¹: C₁-C₄-Alkyl wie Methyl, Ethyl, n-Propyl, 1-Methylethyl, n-Butyl, 1-Methyl-propyl, 2-Methylpropyl und 1,1-Dimethylethyl, vorzugsweise Methyl, Ethyl, n-Propyl, n-Butyl und 1-Methylethyl, insbesondere Methyl und Ethyl;
- R²: Methyl, Ethyl, n-Propyl, 1-Methylethyl, n-Butyl, 1-Methylpropyl, 2-Methylpropyl, 1,1-Dimethylethyl oder Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 2,2-Di-methylpropoxy, 1-Ethylpropoxy, n-Hexoxy, 1,1-Dimethylpropoxy, 1,2-Dimethylpropoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethyl-butoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy,
vorzugsweise Methyl, Ethyl, n-Propyl, n-Butyl und Methoxy, Ethoxy, n-Propoxy und n-Butoxy, insbesondere Methyl, Ethyl, Methoxy und Ethoxy;
oder
- R¹ und R²: zusammen eine C₄-C₆-Alkylenkette wie -CH₂CH₂CH₂CH₂-, -CH₂CH₂CH₂CH₂CH₂- und -CH₂CH₂CH₂CH₂CH₂CH₂-;

- X: Nitro, Cyano;
Fluor, Chlor, Brom und Iod, insbesondere Fluor und Chlor;
C₁-C₄-Alkyl wie Methyl, Ethyl, n-Propyl, 1-Methylethyl, n-Butyl, 1-Methyl-propyl, 2-Methylpropyl, 1,1-Dimethylethyl, vorzugsweise Methyl, Ethyl, n-Propyl oder n-Butyl, insbesondere Methyl oder Ethyl;
C₁-C₄-Halogenalkyl, besonders Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Tri-fluormethyl, Difluormethyl und Fluormethyl;
- Y: Wasserstoff, Nitro, Cyano;
Fluor, Chlor, Brom und Iod, insbesondere Fluor und Chlor;
C₁-C₄-Alkyl wie für X genannt, vorzugsweise Methyl, Ethyl, n-Propyl oder n-Butyl, insbesondere Methyl oder Ethyl;
C₁-C₄-Halogenalkyl, besonders C₁-C₂-Halogenalkyl wie für X genannt, vorzugsweise Trifluormethyl, Difluormethyl und Fluormethyl;
- Alk: 1,2-Ethylen oder 1,3-Propylen, welche beide unsubstituiert sein oder ein bis drei C₁-C₃-Alkylgruppen wie Methyl, Ethyl, n-Propyl und 1-Methylethyl, vorzugsweise Methyl und Ethyl, insbesondere Methyl tragen können.
- Ganz: besonders bevorzugt bedeutet R¹ und R² jeweils C₁-C₄-Alkyl.

Die Hydroxyiminoverbindungen II sind teilweise kommerziell erhältlich oder nach literaturbekannten Methoden herstellbar (vgl. z.B. U.S. 4,743,701).

Die Alkylierungsmittel III sind teilweise bekannt oder können nach literaturbekannten Methoden hergestellt werden.

Die folgenden beiden Reaktionsgleichungen zeigen schematisch einen der möglichen Synthesewege zur Herstellung von Verbindungen III ausgehend von Phenoxyalkansäuren oder deren Estern. Durch Umsetzung mit geeigneten Reduktionsmitteln, beispielsweise Lithiumaluminiumhydrid oder Natriumborhydrid, in einem inerten Lösungsmittel wie Tetrahydrofuran, erhält man Phenoxyalkanole V [vgl. z.B. J. Pharmacol. Chemother. 7, 197 (1952)]:

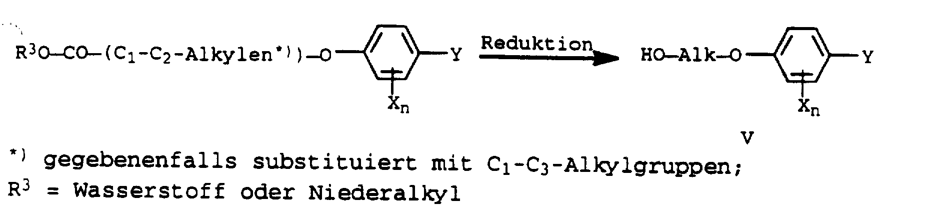

Die Phenoxyalkanole V können dann durch Umsetzung mit anorganischen oder organischen Säurehalogeniden in die Alkylierungsmittel III überführt werden. Beispielsweise erfolgt der Austausch von Hydroxy gegen CH₃-SO₂-O- mit Methansulfonsäurechlorid in Gegenwart eines tertiären Amins:

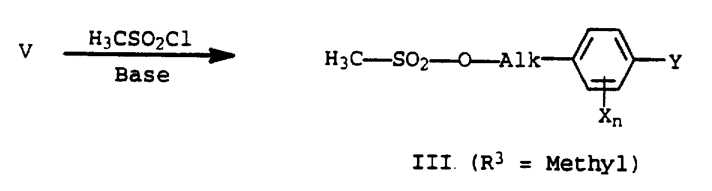

Ersetzt man die Hydroxylgruppe der Phenoxyalkanole V durch Chlor, Brom (z.B. mittels Phosphortribromid in Gegenwart eines tertiären Amins) oder Jod, so erhält man die halogenierten Alkylierungsmittel VI, die anstelle der Alkylierungsmittel III verwendet werden können:

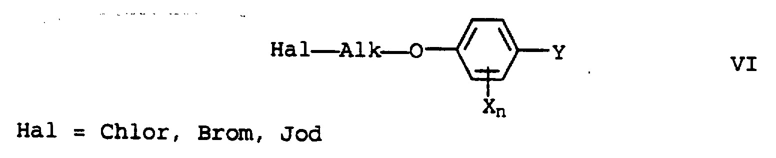

Die Umsetzung von II mit III wird im einzelnen wie folgt durchgeführt:

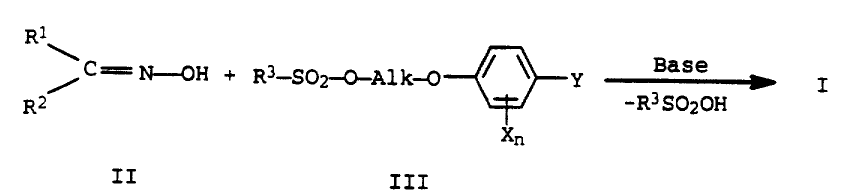

Normalerweise liegt die Reaktionstemperatur bei 20 bis 150°C, vorzugsweise bei 40 bis 120°C, insbesondere bei 60 bis 100°C.

Als Basen kommen beispielsweise Alkalimetallhydroxide wie Natriumhydroxid und Kaliumhydroxid, Alkalimetallalkoholate wie Lithiummethylat, Natriummethylat, Kaliummethylat, Lithiumethylat, Natriumethylat, Kaliumethylat, Natrium-tert.-butylat und Kalium-tert.butylat, Alkalimetallcarbonate wie Natriumcarbonat und Kaliumcarbonat, Alkalimetallhydrogencarbonate wie Natriumhydrogencarbonat und Kaliumhydrogencarbonat, in Betracht.

Besonders bevorzugt sind die vorstehend genannten Natriumverbindungen, insbesondere Natriumhydroxid und Natriummethylat.

Die Base wird zweckmäßigerweise in äquivalenter Menge, bezogen auf die Verbindung II, eingesetzt.

Bezüglich der Definition dipolar-aprotischer Lösemittel sei auf Chr. Reichardt, Lösungsmittel-Effekte in der organischen Chemie, Verlag Chemie 1969, verwiesen. Insbesondere sind als dipolar-aprotische Lösungsmittel solche Lösungsmittel zu verstehen, die keine Wasserstoffbrücken-Donatoren sind und ein ausgeprägtes Dipolmoment (µ größer als 2,5 Debeye) sowie eine hohe Dielektrizitätskonstante (ε größer als 15) aufweisen.

Geeignete dipolar-aprotische Lösungsmittel sind beispielsweise Sulfoxide wie Dimethylsulfoxid, Diäthylsulfoxid, Dimethylsulfon, Diäthylsulfon, Methyläthylsulfon, Tetramethylensulfon; Nitrile wie Acetonitril, Benzonitril, Butyronitril, Isobutyronitril, m-Chlorbenzonitril; N,N-disubstituierte Carbonsäureamide wie Dimethylformamid, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid, Carbonsäurepyrrolidid; entsprechende N,N-Diäthyl-, N,N-Di-n-propyl-, N,N-Diisopropyl-, N,N-Diisobutyl-, N,N-Dibenzyl-, N-Methyl-N-phenyl-, N-Cyclohexyl-N-methylcarbonsäureamide, N-Me-thyl-formanilid; N-Alkyllactame wie N-Äthylpyrrolidon, N-Octylpyrrolidon, N-Cyclohexylpyrrolidon, N-Methylpyrrolidon, N-Butylpyrrolidon; tetrasubstituierte cyclische und acyclische Harnstoffe wie Tetramethylharnstoff, Tetrabutylharnstoff, 1,3-Dimethyl-2-imidazolinon, 1,3-Dimethyl-3,4,5,6-tetrahydro-2(1H)-pyrimidon und Mischungen der genannten Lösungsmittel. Bevorzugt werden N,N-Dialkyl-substiuierte Carbonsäureamide wie Dimethylformamid und Dimethylacetamid oder N-Alkyl-substituierte Lactame wie N-Methylpyrrolidon.

Das Lösungsmittel bzw. Lösungsmittelgemisch wird im allgemeinen in einer Menge von 0,3 bis 1,0 1, vorzugsweise 0,4 bis 0,8 1, insbesondere 0,5 bis 0,7 1, pro mol an Hydroxyiminoverbindung II, eingesetzt.

Die Edukte II und III werden im allgemeinen in äquimolaren Mengen eingesetzt, jedoch kann es zur Optimierung der Ausbeute vorteilhaft sein, II in einem Überschuß von 0,1 bis 0,5 mol-äq., vorzugsweise 0,2 bis 0,4 mol-äq., insbesondere 0,2 bis 0,3 mol-äq., bezogen auf III einzusetzen.

Nach Beendigung der Reaktion kann mittels Destillation bei vermindertem Druck der überwiegende Teil des eingesetzten Lösungsmittels zurückgewonnen werden. Nach Versetzen des Rückstandes bei Raumtemperatur mit Wasser können die Verfahrensprodukte IV abgetrennt werden, gewünschtenfalls durch Extraktion mit beispielsweise Kohlenwasserstoffen wie Toluol und Cyclohexan. Sollen die Oximino-Derivate IV in reiner Form dargestellt werden, so kann eine Reinigung der Rohprodukte auf an sich bekannte Weise, z.B. durch Kristallisation oder fraktionierter Destillation unter reduziertem Druck, erfolgen.

Für die Umsetzung selbst wird zweckmäßigerweise zunächst eine Lösung der Hydroxyiminoverbindung II hergestellt, die Base zugefügt, diese Mischung danach auf die Reaktionstemperatur gebracht, einige Zeit zur Salzbildung nachgerührt und dann das Alkylierungsmittel III, gewünschtenfalls in Lösung, zugefügt.

Vor Zugabe des Alkylierungsmittels III kann es vorteilhaft sein, den durch die Salzbildung freigesetzten Alkohol bzw. das freigesetzte Wasser durch Andestillieren unter vermindertem Druck abzutrennen. Dies wäre ganz besonders bei Verwendung der Alkylierungsmitteln VI anstelle von III zu empfehlen.

Die Hydroxyiminoverbindung der Formel II kann in einem vorgelagerten Schritt auch in ihr Alkalimetallsalz überführt und gewünschtenfalls als solches isoliert werden. Dieses wird dann mit dem für die Umsetzung gewählten Lösungsmittel versetzt und ohne weitere Hilfsbasen mit dem Alkylierungsmittel III zur Reaktion gebracht. Hierzu eignen sich vorteilhafterweise die zuvor genannten Alkalimetallcarbonate, -hydrogencarbonate, -hydroxide und -alkoholate. Diese werden in dafür üblichen Lösungsmitteln, z.B. in Alkoholen, oder in Wasser in stöchiometrischen Mengen mit der betreffenden Hydroxyiminoverbindung II zwischen 0 und 50°C umgesetzt. Besonders bewährt hat sich hierbei das Arbeiten mit Natriummethylatlösung, gegebenenfalls unter Zusatz von Kohlenwasserstoffen wie Toluol. Zweckmäßigerweise werden nach kurzzeitigem Nachrühren (10-60 min) die leichtflüchtigen Anteile entfernt, üblicherweise unter reduziertem Druck. Der Rückstand enthält das Alkalimetallsalz von II.

Obwohl die Herstellung der Oximino-Derivate IV prinzipiell auch aus II - bzw. dem entsprechenden Anion von II - und einem Alkylierungsmittel VI möglich ist, haben sich die Alkylierungsmittel III besonders bewährt. R³ bedeutet hierbei vorzugsweise C₁-C₄-Alkyl, C₁-C₄-Halogenalkyl, Phenyl oder durch Halogen und/ oder C₁-C₄-Alkyl ein- bis dreifach substituiertes Phenyl. Ganz besonders bevorzugt sind CH₃-SO₂-O-, C₆H₅-SO₂-O-, (4-CH₃-C₆H₄)-SO₂-O- oder [2,4,6-(CH₃)₃-C₆H₂]-SO₂-O-.

Bei denjenigen Hydroxyiminoverbindungen II, bei denen R² für C₁-C₆-Alkoxy steht, ist von besonderem Vorteil, daß bei Verwendung von Alkalimetallalkoholaten als Basen der durch die Salzbildung zu Beginn oder während der Reaktion freigesetzte Alkohol in der Reaktionsmischung verbleiben kann. Unerwünschte Nebenreaktionen wie die Eliminierung von R³-SO₂-OH oder die Etherbildung zwischen dem Alkohol und III, werden dadurch sehr stark unterdrückt.

Bei denjenigen Hydroxyiminoverbindungen II dagegen, bei denen R² für C₁-C₆-Alkyl steht, ist die Verwendung von Alkalimetallhydroxiden als Basen und N-Alkylpyrrolidonen, vorzugsweise N-Methylpyrrolidon, als Lösungsmittel besonders vorteilhaft, da in diesen Fällen das durch die Salzbildung zu Beginn oder während der Reaktion freigesetzte Wasser in der Reaktionsmischung verbleiben kann. Bei dieser Verfahrensführung werden unerwünschte Nebenreaktionen wie Eliminierung von R³-SO₂-OH oder Verseifung des Alkylierungsmittels III sehr stark unterdrückt.

Aus IV läßt sich durch saure Hydrolyse der entsprechende Hydroxylamin-Ether der Formel I freisetzen. I fällt dabei zunächst in Form des Salzes der verwendeten Säure an und kann als solches oder, nach Zugabe von Base, als freier Hydroxylamin-Ether I isoliert werden.

Für die Spaltung haben sich Mineralsäuren, vorzugsweise Salzsäure und Phosphorsäure, und starke organische Säuren wie Trichloressigsäure, Trifluoressigsäure als geeignet erwiesen. Diejenigen Oximino-Derivate IV, in denen R² für C₁-C₄-Alkyl steht, lassen sich besonders vorteilhaft mit Mineralsäuren spalten.

Unter den Mineralsäuren ist Salzsäure, der gewünschtenfalls ein Kosolvens zugesetzt werden kann, ganz besonders bevozugt. Als Kosolventien sind z.B. Alkohole geeignet.

Normalerweise erfolgt die Spaltung bei einer Temperatur von 50 bis 120°C mit ausreichender Geschwindigkeit.

Die Menge an Säure ist nicht kritisch. Für eine vollständige Hydrolyse benötigt man mindestens die äquivalente Menge an Säure, bezogen auf IV. Im allgemeinen sind 1 bis 10 mol Säure pro mol IV oder, falls IV nicht isoliert wird, pro mol II oder III ausreichend. Eine höhere Säuremenge ist auch möglich, bietet üblicherweise aber keine Vorteile.

Oximino-Derivate der Formel IV, in denen R¹ für Methyl und R² für Ethoxy steht, lassen sich auch analog der in der DE-A 26 51 083 angegebenen Methode hydrolysieren.

In der Regel können alle genannten Verfahrensschritte bei Atmosphärendruck oder unter dem Eigendruck des jeweiligen Systems ausgeführt werden.

Nach dem vorliegenden Verfahren sind die Hydroxylamin-Ether I auf technisch einfache Weise erhältlich. Besonders vorteilhaft ist hierbei, daß bei der Spaltung der Oximino-Derivate IV neben den Hydroxylamin-Ethern I weitere Wertprodukte anfallen, nämlich die Folgeprodukte des Schutzgruppenteils (also Ketone oder Ester). In vielen Fällen kann sogar die Schutzgruppe zurückgewonnen und erneut zur Herstellung der Hydroxyiminoverbindungen II eingesetzt werden. So ist beispielsweise im Falle R¹ und R² = Methyl die Rückführung des bei der Hydrolyse gebildeten Acetons zur erneuten Herstellung von Acetonoxim II (R¹, R² = Methyl) möglich.

Die Hydroxylamin-Ether der Formel I sind wichtige Zwischenprodukte für Pflanzenschutzmittel und Pharmaka. Sie können, als freie Basen oder als Salze, beispielsweise in an sich bekannter Weise mit Cyclohexantrionen oder Pyronen VII zu den entsprechenden Oximethern VIII kondensiert werden, die vorzugsweise im Pflanzenschutz als Herbizide Verwendung finden (vgl. z.B. EP-A 136 702, EP-A 142 741 und EP-A 456 112):

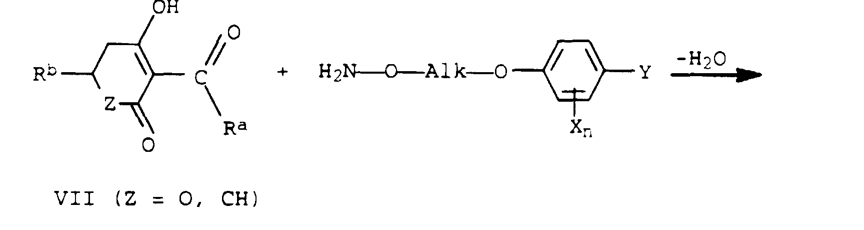

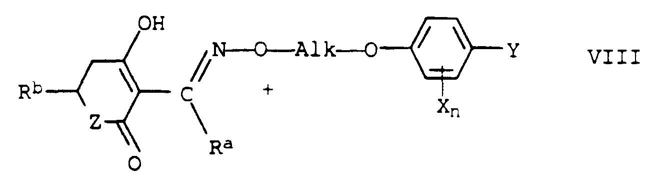

R^{a} bedeutet vorzugsweise C₁-C₄-Alkyl und R^{b} steht z.B. für Alkoxyalkyl, Alkylthioalkyl, eine gegebenenfalls substituierte Cycloalkyl- oder Cycloalkenylgruppe, einen gegebenenfalls substituierten 5-gliedrigen Heterocyclus oder Heteroaromat, einen gegebenenfalls substituierten 6- oder 7-gliedrigen Heterocyclus oder einen gegebenenfalls substituierten Phenyl- oder Pyridylring.

### Herstellungsbeispiele

### Beispiel 1

### 2-(4-Chlorphenoxy)-1-(1-ethoxyethylidenaminooxy)-propan {= O-[2-(4-Chlorphenoxy)propyl]acethydroximsaureethylester} (Tabelle 1, Verbindung Nr. 18)

a) Alkylierung mit Kaliummethylat als Base
   Zu 155 g (1,5 mol) Acethydroximsäureethylester in 1500 ml absolutem Dimethylformamid wurden 105,2 g (1,5 mol) Kaliummethylat gegeben (leicht exotherme Reaktion). Nach Rühren für 45 min bei 25-30°C erhielt man eine klare Lösung, die innerhalb von 2,5 Std. zu einer 50°C warmen Lösung von 265 g (1 mol) 2-(4-Chlorphenoxy)propyl-methan-sulfonat in 600 ml Dimethylformamid gegeben wurde. Nach vollständiger Zugabe rührte man noch 4 Std. bei 50°C, wonach noch l Std. auf 100°C erhitzt und dann abgekühlt wurde. Anschließend entfernte man das Dimethylformamid im Wasserstrahlvakuum bei einer Badtemperatur von max. 100°C. Nach dem Abkühlen des Rückstandes nahm man mit 1 l Toluol und 1 l 1 gew.-%iger Natronlauge auf. Die wäßrige Phase wurde abgetrennt und einmal mit 200 ml Toluol extrahiert. Die vereinigten organischen Phasen wurden zweimal mit je 200 ml 1 gew.-%iger Natronlauge und einmal mit Wasser gewaschen, getrocknet und eingeengt. Das erhaltene Rohprodukt eignete sich bei einer Reinheit von 94,4% (GC-Flächenprozent) direkt für die weitere Umsetzung (Freisetzung des Hydroxylaminethers).
   Gewünschtenfalls kann das Produkt mittels fraktionierter Destillation bei reduziertem Druck gereinigt werden.
   Ausbeute: 89%; Kp. 100-101°C (bei 0,2 mbar)
b) Alkylierung mit Natriummethylat als Base
   Zu 30,9 g (0,3 mol) Acethydroximsäureethylester in 450 ml Dimethylformamid tropfte man bei 50°C 54 g 30 gew.-%ige methanolische Natriummethylatlösung (0,3 mol Natriummethylat). Die erhaltene Mischung wurde noch 30 min gerührt, wonach man unter reduziertem Druck bei max. 50°C Innentemperatur 180 ml Flüssigkeit aus dem Reaktionsgemisch abdestillierte. Zum Konzentrat tropfte man innerhalb von 40 min bei gleicher Temperatur 52,9 g (0,2 mol) 2-(4-Chlorphenoxy)propyl-methansulfonat gelöst in 70 ml Dimethylformamid. Nach weiteren 18 Std. Rühren bei 50°C wurde wie unter a) beschrieben aufgearbeitet.
   Man erhielt die Titelverbindung mit 79%iger Ausbeute.

### Beispiel 2

### 2-(4-Chlorphenoxy)-1-isopropylidenaminooxy-propan {= Aceton-[0-2-(4-chlorphenoxy)propyl]-oxim} (Tabelle 1, Verbindung Nr.28)

a) Alkylierung mit Natriumhydroxid als Base
   Zu 68,3 g (0,94 mol) Acetonoxim und 306 ml N-Methylpyrrolidon wurden unter Rühren 37,4 g (0,94 mol) Natriumhydroxid gegeben. Man erwärmte auf 100°C Innentemperatur und tropfte innerhalb von 45 min 24,5 g (0,85 mol) 2-(4-Chlorphenoxy)propyl-methansulfonat, gelöst in 155 ml N-Methylpyrrolidon, zu. Nach 2 Std. wurde die Reaktionsmischung auf 300C abgekühlt, wonach man unter reduziertem Druck bei einem Siedepunkt von 46°C (2 mbar) 415 g N-Methylpyrrolidon abdestillierte, das in das Verfahren zurückgeführt werden kann. Anschließend ließ man abkühlen, fügte 500 ml Wasser zu, rührte 45 min und extrahierte fünfmal mit je 250 ml Cyclohexan. Nach Trocknen und Einengen wurde das Rohprodukt mittels fraktionierter Destillation gereinigt.
   Man erhielt die Titelverbindung mit 80%iger Ausbeute; Kp. 83-87°C (bei 0,1 mbar).
b) Alkylierung mit Acetonoximnatriumsalz
   Eine 30 gew.-%ige methanolische Natriummethylat-Lösung wurde mit dem 3fachen Volumen an Toluol verdünnt, wonach die äquivalente Menge an Acetonoxim eingetragen wurde. Danach entfernte man die niedrig siedenden Anteile unter reduziertem Druck.
   Zu 142,6 g (1,5 mol) Acetonoximnatriumsalz, vorgelegt in 490 ml N-Methylpyrrolidon, gab man bei 100°C tropfenweise 264,1 g (1 mol) 2-(4-Chlorphenoxy)propyl-methansulfonat, gelöst in 180 ml N-Methylpyrrolidon. Danach ließ man noch 1 Std. nachreagieren und arbeitete danach wie oben beschrieben auf.
   Man erhielt 590 ml N-Methylpyrrolidon zurück. Die Ausbeute an Titelverbindung betrug 81 % (Reinheit nach GC 96%).

In Tabelle 1 sind weitere Oximino-Derivate IV aufgeführt, die auf die gleiche Weise hergestellt wurden oder herstellbar sind.

### Beispiel 3

### 1-Aminooxy-2-(4-chlorphenoxy)propan {= 2-(4-Chlorphenoxy)propoxyamin}

a) Hydrolyse von O-[2-(4-Chlorphenoxy)propyl]acethydroximsäureethylester
   Zu 1,7 l (3,4 mol) 2n Salzsäure tropfte man bei 20-25°C innerhalb von 60 min 485 g (1,7 mol) O-[2-(4-Chlorphenoxy)propyl]acethydroximsäureethylester (Tabelle 1, Verbindung Nr. 18; 95%ig rein nach GC) und erhitzte die Mischung für 30min auf Rückflußtemperatur. Danach wurde abgekühlt, unter Eisbadkühlung mit 280 ml 50 gew.-%iger Natronlauge auf pH=10 gestellt und dreimal mit je 400 ml Dichlormethan extrahiert. Die vereinten organischen Phasen wurden mit Wasser gewaschen, anschließend getrocknet und eingeengt.
   Man erhielt die Titelverbindung mit einer Ausbeute von 97% (96,9%ig rein nach GC-Analyse).
   Gewünschtenfalls kann die Verbindung destillativ gereinigt werden; Kp. 102-104°C (0,4 mbar).
   250-MHz-¹H-NMR (in CDCl₃): δ [ppm] = 1,25 (d,3H; CH₃); 3,6-3,9 (m,2H; -O-CH₂); 4,64 (m,1H; Ph-O-CH(CH₃)); 5,5 (breites s,2H; NH₂); 6,9 und 7,2 (2d,4H; Ph-H)
b) Hydrolyse von Aceton- [O-2-(4-chlorphenoxy)propyl]-oxim mit Trichloressigsäure
   In einer Rührapparatur mit aufgesetzter 30-cm-Kolonne wurde eine Mischung aus 10 g (4,13 mmol) Aceton-[O-2-(4-chlorphenoxy)propyl]-oxim (Verbindung 28 in Tabelle 1) und 44 g 30gew.-%iger wäßriger Trichloressigsäurelösung bei 78°C und 450 mbar Unterdruck 10 Std. lang erhitzt, wobei kontinuierlich 110 g Wasser zu der Reaktionsmischung getropft wurden, bei kontinuierlicher Abdestillation des entstehenden Wasser/ Aceton-Gemisches. Zur Aufarbeitung wurde der Reaktionsaustrag mit 10 gew.-%iger Natronlauge alkalisch gestellt und mit Toluol extrahiert. Man isolierte 6 g 2-(4-Chlorphenoxy)propoxyamin. Ausbeute: 72%.
c) Hydrolyse von Aceton-O-2-[4-chlorphenoxy)propyl]-oxim mit Trifluoressigsäure
   Analog Versuch 3b) wurden 10 g (4,13 mmol) Aceton-[O-2-(4-chlorphenoxy)propyl]-oxim und 31 g 30 %ige wäßrige Trifluoressigsäure-Lösung bei 430 mbar Unterdruck 8³/4 Std. auf 80°C erhitzt, wobei kontinuierlich 100 g Wasser zu der Reaktionsmischung getropft wurden, bei Abdestillation eines Wasser/Aceton-Gemisches. Die zu 3b) analoge Aufarbeitung liefert hier 6,4 g 2-(4-Chlorphenoxy)propoxyamin. Ausbeute: 76%.
d) Hydrolyse von Aceton-O-2-[4-chlorphenoxy)propyl]-oxim mit Salzsäure
   In einer Rührapparatur wurden 10 g (4,13 mmol) Aceton-[O-2-(4-chlorphenoxy)propyl]-oxim in einer Mischung aus 250 g n-Propanol, 38 g konzentrierter Salzsäure (38 gew.-%ig) und 60 g Wasser gelöst. Diese Lösung erhitzte man 6 Std. auf 80°C und destillierte anschließend zur Aufarbeitung n-Propanol und Wasser ab. Nach Umkristallisation des Rückstandes aus 20 gew.-%iger Salzsäure erhielt man 7,7 g des Hydrochlorids vom 2-(4-Chlorphenoxy)-propoxyamin. Ausbeute: 78 %.

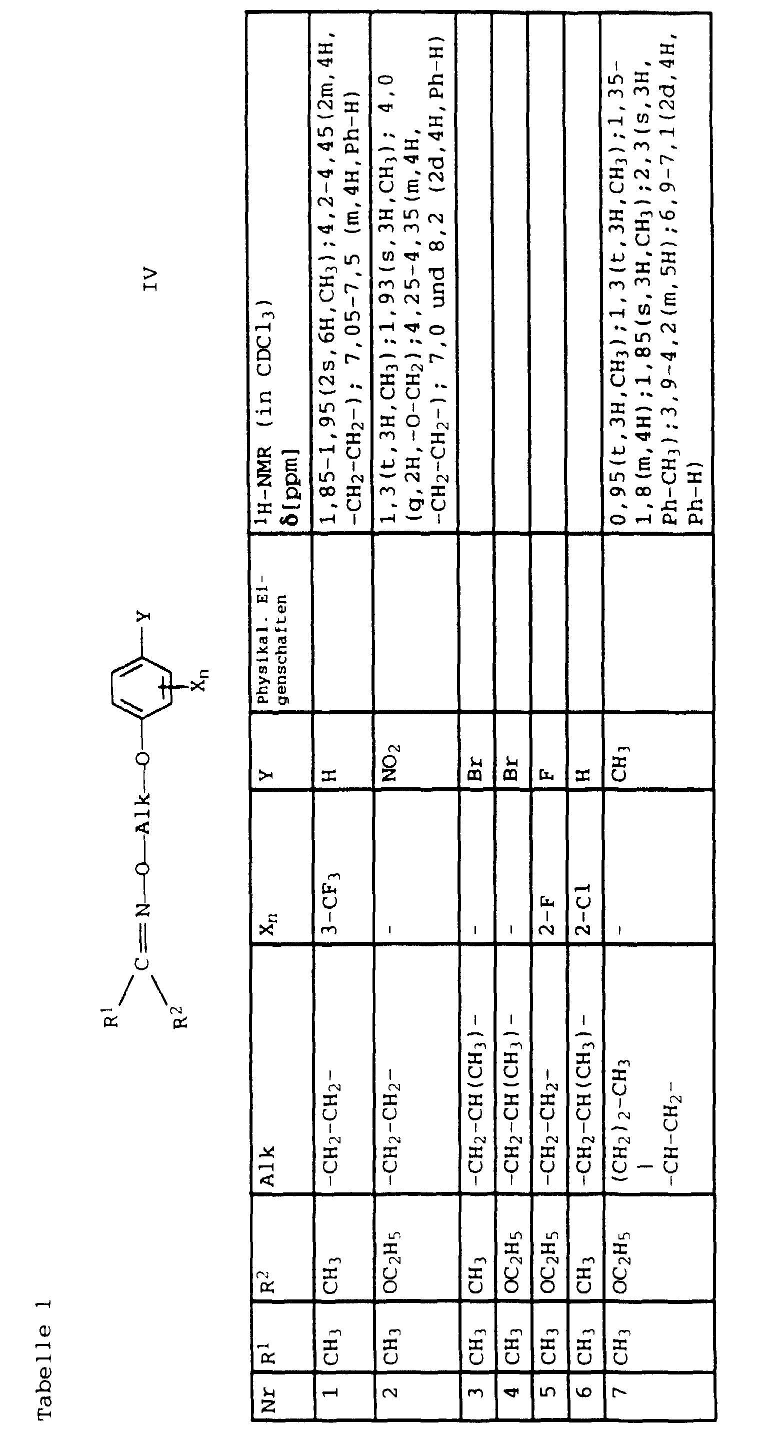

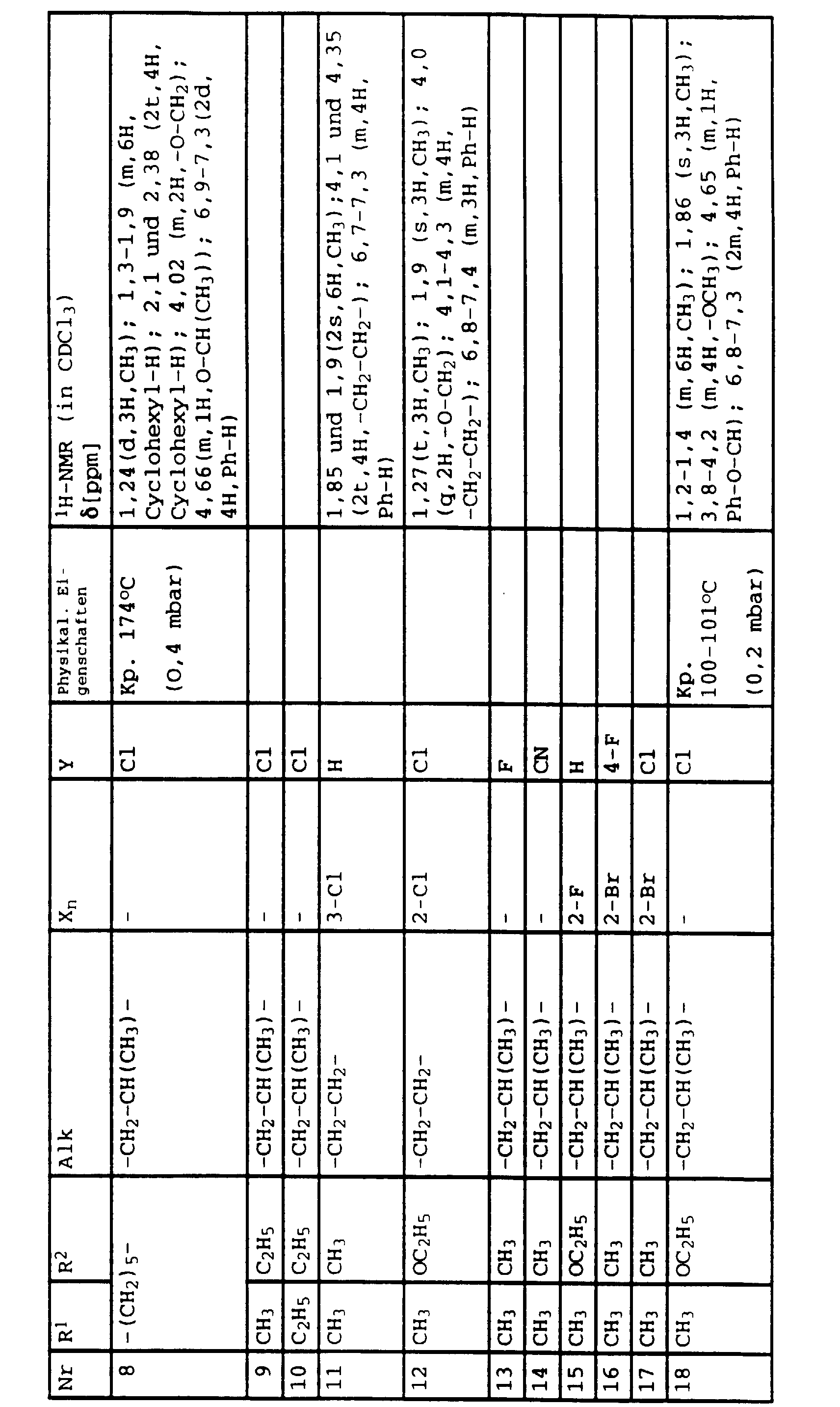

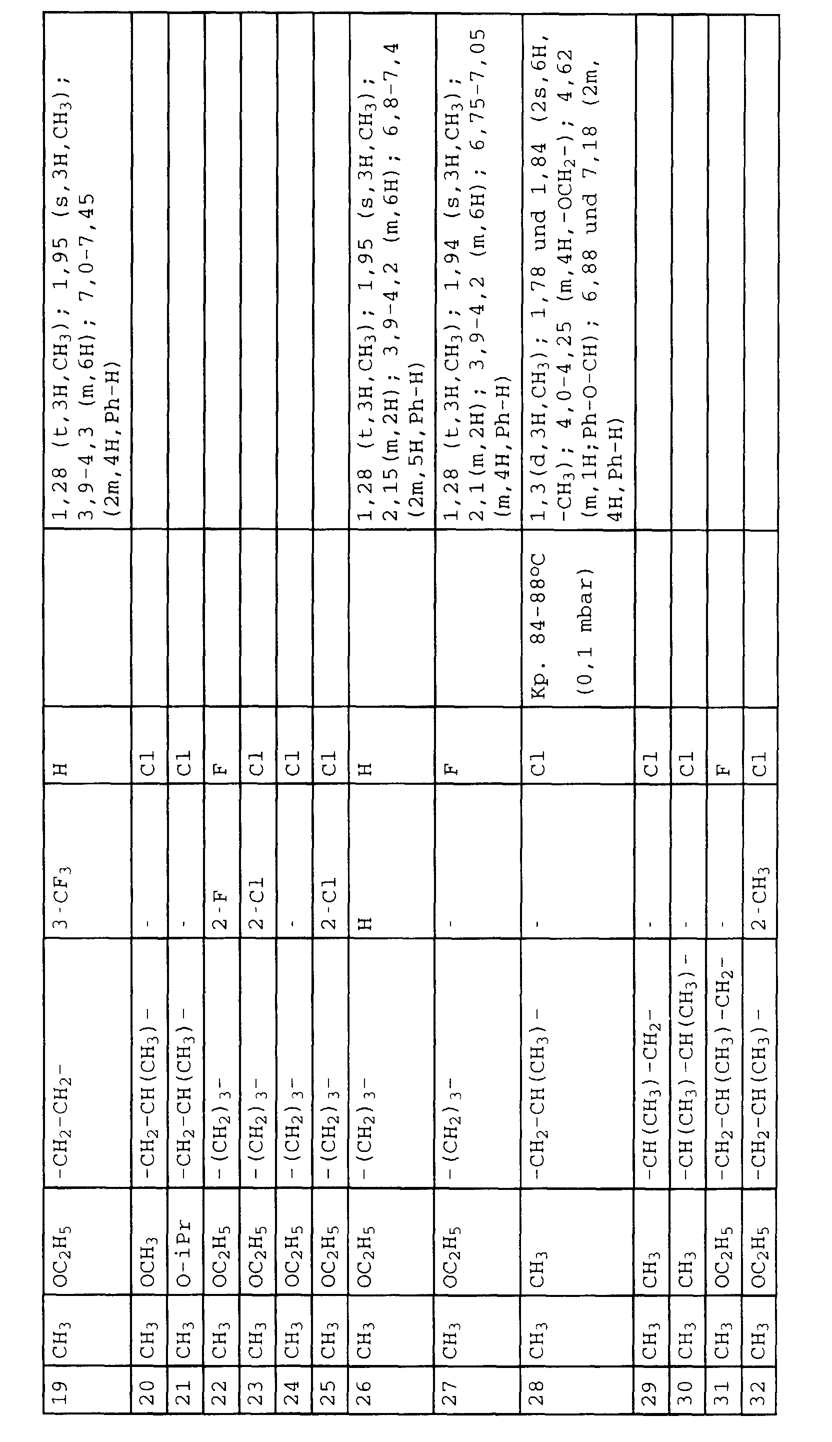

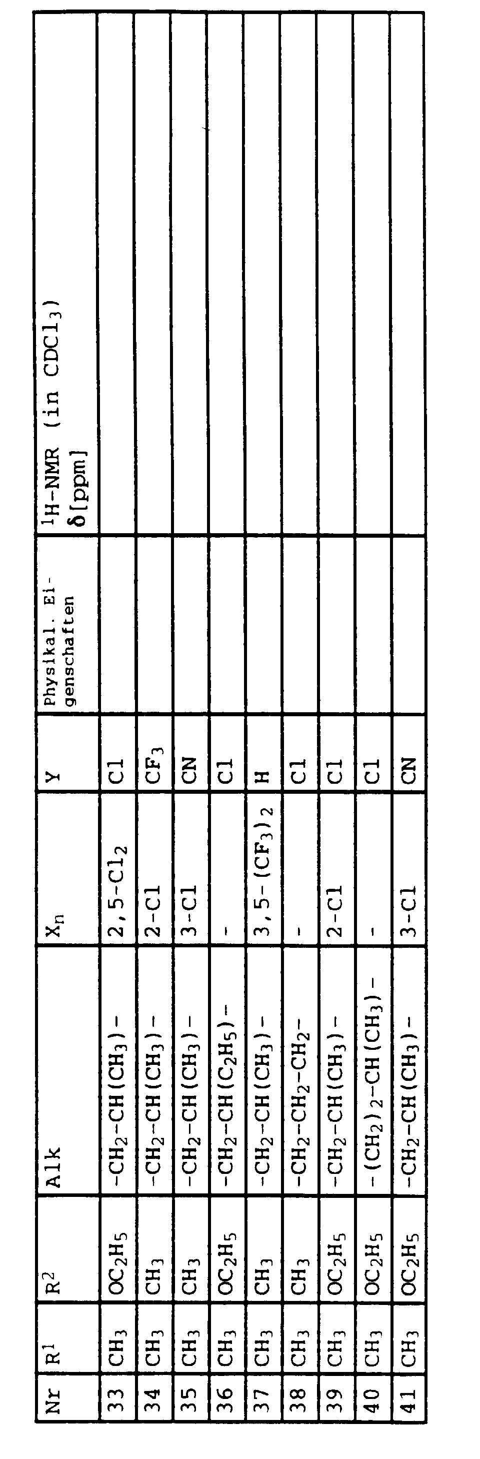

## Patentansprüche

1. Verfahren zur Herstellung von Hydroxylamin-Ethern der Formel I 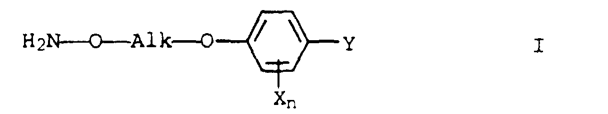 in der die Variablen folgende Bedeutung haben:
X Nitro, Cyano, Halogen, C₁-C₄-Alkyl und C₁-C₄-Halogenalkyl;
Y Wasserstoff, Nitro, Cyano, Halogen, C₁-C₄-Alkyl und C₁-C₄-Halogenalkyl;
n 0-2 oder 1-4 für den Fall, daß Y und alle X Halogen bedeuten;
Alk eine C₂- oder C₃-Alkylenkette, die gewünschtenfalls ein bis drei C₁-C₃-Alkylgruppen tragen kann,
sowie deren Salze mit Mineralsäuren oder starken organischen Säuren,
dadurch gekennzeichnet, daß man entweder eine Hydroxyiminoverbindung der Formel II 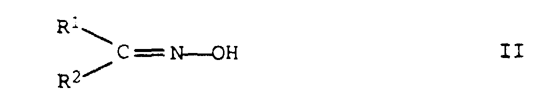 in der R¹ für C₁-C₄-Alkyl, R² für C₁-C₄-Alkyl oder C₁-C₆-Alkoxy oder R¹ und R² zusammen für eine C₄-C₆-Alkylenkette stehen,
in Gegenwart von Alkalimetallhydroxid, Alkalimetallalkoholat, Alkalimetallhydrogencarbonat oder Alkalimetallcarbonat als Base,
oder das entsprechende Anion von II direkt,
mit einem Alkylierungsmittel der Formel III 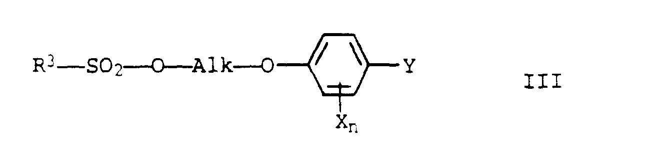 in der R³ für C₁-C₄-Alkyl, C₁-C₄-Halogenalkyl oder gegebenenfalls substituiertes Phenyl steht,
zu einem Oximino-Derivat der Formel IV 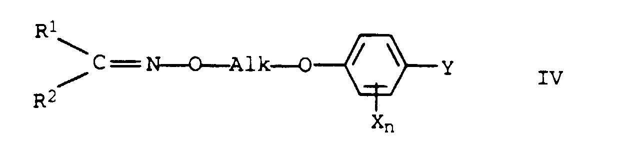 umsetzt,
das Oximino-Derivat IV anschließend mittels Mineralsäure oder einer starken organischen Säure zum entsprechenden Salz von I spaltet und dieses gewünschtenfalls mittels Base in die freie Verbindung I überführt.

2. Verfahren nach Anspruch 1, dadurch gekennzeichnet, daß man die Umsetzung von II mit III in 0,3 bis 1,0 l eines organischen Lösungsmittels pro mol II vornimmt.

3. Verfahren nach Anspruch 1, dadurch gekennzeichnet, daß man die Umsetzung von II mit III in einem dipolar-aprotischen Lösungsmittel, das ein großes Dipolmoment (µ größer als 2,5 Debeye) und eine hohe Dielektrizitätskonstante (ε größer als 15) hat, vornimmt.

4. Verfahren nach Anspruch 1, dadurch gekennzeichnet, daß man die Umsetzung von II mit III in N,N-disubstituierten Carbonsäureamiden oder in N-substituierten Lactamen vornimmt.

5. Verfahren nach Anspruch 1, dadurch gekennzeichnet, daß man die Umsetzung von II mit III in Gegenwart von Natriumhydroxid, Natriumalkoholat, Kaliumalkoholat, Natriumhydrogencarbonat oder Natriumcarbonat als Base vornimmt.

6. Verfahren nach Anspruch 1, dadurch gekennzeichnet, daß man diejenigen Verbindungen II, bei denen R² für C₁-C₄-Alkoxy steht, mit III in Gegenwart eines Alkalimetallalkoholats als Base in einem N,N-Dialkyl-substituierten Carbonsäureamid umsetzt.

7. Verfahren nach Anspruch 1, dadurch gekennzeichnet, daß man diejenigen Verbindungen II, bei denen R² für C₁-C₄-Alkyl steht, mit III in Gegenwart von Alkalimetallhydroxid als Base in einem N-substituierten 2-Pyrrolidon als Lösungsmittel umsetzt.

8. Verfahren nach Anspruch 1, dadurch gekennzeichnet, daß man diejenigen Oximino-Derivate IV, bei denen R² für C₁-C₄-Alkyl steht, mit Mineralsäure spaltet.

## Claims

1. A process for the preparation of hydroxylamine ethers of the formula I 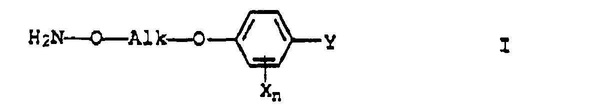 in which the variables have the following meaning:
X is nitro, cyano, halogen, C₁-C₄-alkyl and C₁-C₄-haloalkyl,
Y is hydrogen, nitro, cyano, halogen, C₁-C₄-alkyl and C₁-C₄-haloalkyl,
n is 0-2 or 1-4 where Y and all radicals X are halogen and
Alk is a C₂- or C₃-alkylene chain which, if desired, may carry from one to three C₁-C₃-alkyl groups,
and their salts with mineral acids or strong organic acids,
wherein either a hydroximino compound of the formula II 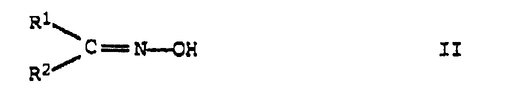 where R¹ is C₁-C₄-alkyl and R² is C₁-C₄-alkyl or C₁-C₆-alkoxy or R¹ and R² together form a C₄-C₆-alkylene chain, in the presence of an alkali metal hydroxide, alkali metal alcoholate, alkali metal bicarbonate or alkali metal carbonate as the base, or the corresponding anion of II directly, is reacted with an alkylating agent of the formula III 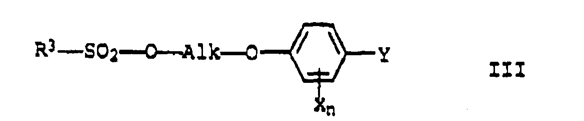 where R³ is C₁-C₄-alkyl, C₁-C₄-haloalkyl or unsubstituted or substituted phenyl, to give an oximino derivative of the formula IV 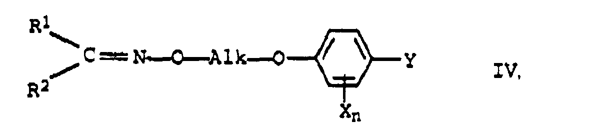 said derivative IV is then cleaved by means of a mineral acid or a strong organic acid to give the corresponding salt of I and, if desired, the latter is converted by means of a base into the free compound I.

2. A process as claimed in claim 1, wherein the reaction of II with III is carried out in from 0.3 to 1.0 1 of an organic solvent per mol of II.

3. A process as claimed in claim 1, wherein the reaction of II with III is carried out in a dipolar aprotic solvent which has a large dipole moment (µ greater than 2.5 Debye) and a high dielectric constant (ε greater than 15).

4. A process as claimed in claim 1, wherein the reaction of II with III is carried out in an N,N-disubstituted carboxamide or in an N-substituted lactam.

5. A process as claimed in claim 1, wherein the reaction of II with III is carried out in the presence of sodium hydroxide, a sodium alcoholate, a potassium alcoholate, sodium bicarbonate or sodium carbonate as the base.

6. A process as claimed in claim 1, wherein those compounds II in which R² is C₁-C₄-alkoxy are reacted with III in the presence of an alkali metal alcoholate as the base in an N,N-dialkyl-substituted carboxamide.

7. A process as claimed in claim 1, wherein those compounds II in which R² is C₁-C₄-alkyl are reacted with III in the presence of an alkali metal hydroxide as the base in an N-substituted 2-pyrrolidone as the solvent.

8. A process as claimed in claim 1, wherein those oximino derivatives IV in which R² is C₁-C₄-alkyl are cleaved with a mineral acid.

## Revendications

1. Procédé pour la préparation d'éthers de l'hydroxylamine répondant à la formule I 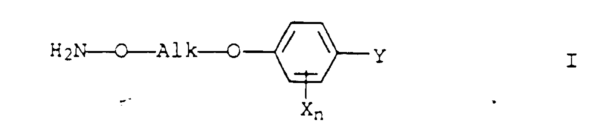 dans laquelle les symboles ont les significations suivantes :
X : un groupe nitro, cyano, un halogène, un groupe alkyle en C1-C4 ou halogénoalkyle en C1-C4 ;
Y : l'hydrogène, un groupe nitro, cyano, un halogène, un groupe alkyle en C1-C4 ou halogénoalkyle en C1-C4 ;
n : 0 à 2 ou 1 à 4 dans les cas où Y et tous les symboles X représentent des halogènes ;
Alk : une chaîne alkylène en C2 ou C3 qui peut le cas échéant porter un à trois groupes alkyle en C1-C3,
et de leurs sels d'acides minéraux ou d'acides organiques forts,
caractérisé par le fait que l'on fait réagir soit un dérivé hydroxyiminé de formule II 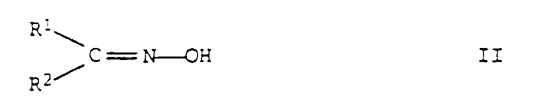 dans laquelle R¹ représente un groupe alkyle en C1-C4, R² un groupe alkyle en C1-C4 ou alcoxy en C1-C6 ou bien R¹ et R², ensemble, une chaîne alkylène en C4-C6, en présence d'un hydroxyde de métal alcalin, d'un alcoolate de métal alcalin, d'un bicarbonate de métal alcalin ou d'un carbonate de métal alcalin, en tant que base, soit l'anion correspondant de II directement,
avec un agent alkylant de formule III 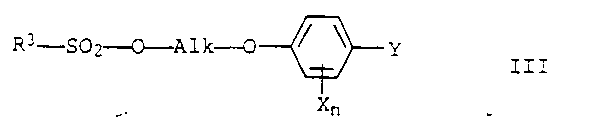 dans laquelle R³ représente un groupe alkyle en C1-C4, halogénoalkyle en C1-C4 ou un groupe phényle éventuellement substitué,
la réaction donnant un dérivé oximiné de formule IV 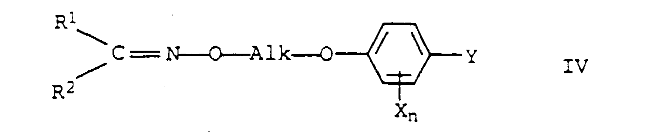 qu'on scinde ensuite à l'aide d'un acide minéral ou d'un acide organique fort en le sel correspondant de I, lequel, si on le désire, peut être converti en le composé I libre à l'aide d'une base.

2. Procédé selon la revendication 1, caractérisé par le fait que la réaction entre II et III est réalisée dans un solvant organique en quantité de 0,3 à 1,0 1 par mol de II.

3. Procédé selon la revendication 1, caractérisé par le fait que la réaction entre II et III est réalisée dans un solvant aprotonique dipolaire à fort moment dipolaire à fort moment dipolaire (µ supérieur à 2,5 debyes) et forte constante diélectrique (ε supérieur à 15).

4. Procédé selon la revendication 1, caractérisé par le fait que la réaction entre II et III est réalisée dans des carboxamides N,N-disubstitués ou dans des lactames N-substitués.

5. Procédé selon la revendication 1, caractérisé par le fait que la réaction entre II et III est réalisée en présence d'hydroxyde de sodium, d'un alcoolate de sodium, d'un alcoolate de potassium, du bicarbonate de sodium ou du carbonate de sodium servant de base.

6. Procédé selon la revendication 1, caractérisé par le fait que les composés II pour lesquels R² représente un groupe alcoxy en C1-C4 sont mis à réagir avec III en présence d'un alcoolate de métal alcalin servant de base et dans un carboxamide N,N-dialkylsubstitué.

7. Procédé selon la revendication 1, caractérisé par le fait que les composés II pour lesquels R² représente un groupe alkyle en C1-C4 sont mis à réagir avec III en présence d'un hydroxyde de métal alcalin servant de base et dans une 2-pyrrolidone substituée à l'azote qui sert de solvant.

8. Procédé selon la revendication 1, caractérisé par le fait que les dérivés oximinés IV pour lesquels R² représente un groupe alkyle en C1-C4 sont scindés à l'aide d'un acide minéral.
